# EUROPÄISCHE PATENTSCHRIFT

(11) **EP 0 867 443 B1**
(45) Veröffentlichungstag und Bekanntmachung des Hinweises auf die Patenterteilung: **06.08.2003**
(21) Anmeldenummer: 98250089.4
(22) Anmeldetag: 13.03.1998
(51) Int. Cl.: C07F 7/18, C08G 77/58, A61K 6/00, A61K 6/093, C08G 77/22

(54) **Hydrolysierbare und polymerisierbare Oxetansilane**
Hydrolyzable and polymerizable oxetanesilanes
Oxetanesilanes hydrolysables et polymérisables

(30) Priorität: 25.03.1997 DE 19714324
(43) Veröffentlichungstag der Anmeldung: 30.09.1998
(73) Patentinhaber: Ivoclar Vivadent AG, 9494 Schaan (LI)
(72) Erfinder: Moszner, Norbert, Prof. Dr., FL-9492 Eschen (LI); Völkel, Thomas, Dr., 88131 Lindau (DE); Stein, Sabine, 6710 Nenzing (AT); Rheinberger, Volker, Dr., 9490 Vaduz (LI)
(74) Vertreter: UEXKÜLL & STOLBERG

(56) Entgegenhaltungen:
- DE-A- 4 133 494
- GB-A- 1 036 077
- GB-A- 1 058 385
- US-A- 5 463 084
- LECAMP, L. ET AL: "Cationic photocrosslinkable polydimethylsiloxane. III. synthesis and photocrosslinking of polydimethylsiloxane bearing oxetane groups" J. MACROMOL. SCI., PURE APPL. CHEM. (1997), A34(11), 2335-2353 , Seiten 2335-2353, XP000905425
- LECAMP, LAURENCE ET AL: "Photopolymerizable polydimethylsiloxanes by cationic methods. II. Synthesis of silanes bearing heterocyclic, or olefinic functions" EUR. POLYM. J. , Bd. 33, Nr. 7, 1997, Seiten 1021-1029, XP004125370

## Beschreibung

Die Erfindung betrifft hydrolysierbare und polymerisierbare Oxetansilane, Verfahren zu ihrer Herstellung, aus ihnen hergestellte Kieselsäurekondensate, Polymerisate und Zusammensetzungen sowie die Verwendung all dieser Materialien unter anderem zur Herstellung von makromolekularen Massen durch Polymerisation und zur Herstellung von Verbundmaterialien, Adhäsiven, Beschichtungen und insbesondere von Dentalmaterialien.

Hydrolysierbare Silane, die polymerisierbare organische Reste enthalten, finden bei der Herstellung von Beschichtungen, partikulären Füllstoffen, Klebemassen und monolithischen Formkörpern sowie bei der Oberflächenmodifizierung von Verstärkungsstoffen Anwendung. Dabei werden die Silane alleine, in Mischung mit anderen Silanen oder in Gegenwart von anderen Metallalkoxiden hydrolytisch kondensiert und thermisch, photochemisch oder redoxinitiiert polymerisiert, d.h. gehärtet.

Im Zusammenhang mit der Herstellung von organisch-anorganischen Verbundmaterialien sind vor allem organisch-modifizierte Silane mit polymerisationsfähigen organischen Gruppen, wie Vinyl-, (Meth)acryl-, Allyl- oder Styryl-Gruppen von besonderem Interesse, da sie den simultanen oder konsekutiven Aufbau sowohl eines anorganischen als auch eines organischen Netzwerkes und damit von Verbundmaterialien mit maßgeschneiderten Eigenschaften gestatten (vgl. H. Schmidt, Mat. Res. Soc. Symp. Proc. Vol. 32 (1984), 327-335; H. Schmidt, H. Wolter, J. Non-Cryst. Solids 121 (1990), 428-435). Dabei werden die polymerisationsfähigen Silane in der Regel zunächst in Lösung hydrolytisch kondensiert. Nach Zugabe von thermischem Initiator oder Photoinitiator und Abtrennung des Lösungsmittels bilden sich dann nanopartikuläre Harze, die nach Formgebung polymerisiert und damit gehärtet werden.

Ein wesentlicher Nachteil dieser Materialien besteht jedoch darin, daß die bei der Polymerisation erfolgende Ausbildung des organischen Netzwerkes meist von einer beträchtlichen Volumenkontraktion begleitet ist, die zur Deformation der Formkörper, zur Verminderung der Substrathaftung, zur Schichtentrennung, zur Ausbildung von Hohlräumen oder zur Ausbildung von Materialspannungen führen kann. Eine verringerte Volumenkontraktion erfolgt bei Silanen, die ringöffnende Gruppen tragen. In diesem Zusammenhang beschreiben die EP-B-0 358 011 kratzfeste Materialien u.a. auf der Basis von 3-Glycidyloxypropylsilanen, die EP-B-0 486 469 organisch-anorganische Hybridpolymere von 3-Glycidyloxypropylsilanen und die DE-C-41 33 494 Dentalharzmassen, bei denen z.B. Silane mit ringöffnenden Spiroorthoestergruppen eingesetzt werden. Dabei erweist es sich allerdings als nachteilig, daß Epoxid-Silane toxikologisch bedenklich sind und erst bei erhöhten Temperaturen hinreichend schnell kationisch polymerisieren. Weiter zeigen Spiroorthoestersilane eine nur geringe Stabilität und ihre kationische Ringöffnungspolymerisation wird in der Regel von einer Lacton-Bildung begleitet.

Weiter sind auch die folgenden siliciumhaltigen Oxetan-Derivate bekannt:
1. Siliconhaltige Oxetane, die z.B. durch Hydrosilylierung von 3-Allyloxymethyl-3-ethyl-oxetan mit 1,1,3,3-Tetramethyldisiloxan zugänglich sind (vgl. J. V. Crivello et al., J. Macromol. Sci.-Pure Appl. Chem. **A30** (1993), 173-187): 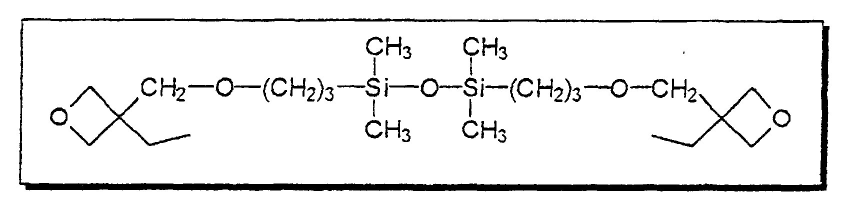
2. 3-(Trimethylsiloxy)-oxetane, die durch Paterno-Büchi-Reaktion synthetisiert werden können (vgl. T. Bach, Tetrahedron Lett. 32 (1991), 7037-8): 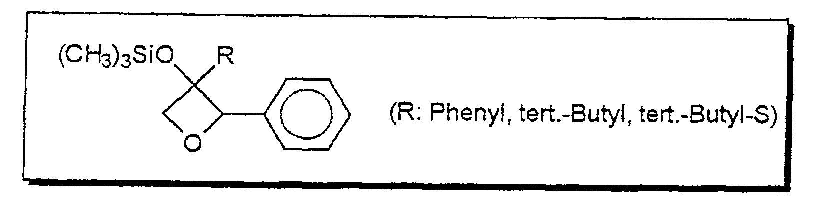
3. 3-Alkyl-3-(triorganosiloxymethyl)-oxetane oder 3-Alkyl-3-(triorganosilylmethyl)-oxetane (vgl. DE-A-195 06 222): 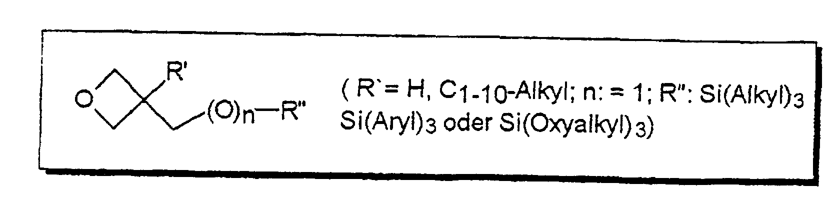
4. 3,3-Bis(triorganosiloxymethyl)-oxetane, die durch Umsetzung von 3,3-Bis(hydroxymethyl)-oxetanen mit entsprechenden Triorganoaminosiloxanen R₃SiNH₂ zugänglich sind (vgl. Chem. Abstr. 76 (192) 14701k):

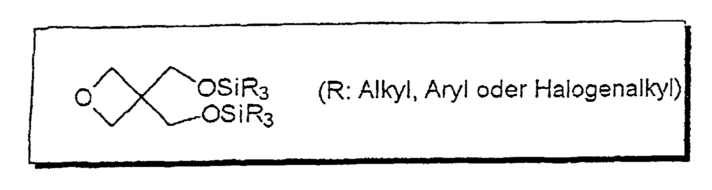

Die GB-A-1 036 077 beschreibt Oxetansilane und Siloxane, die als Schutzbeschichtungen, als Kupplungsmittel für Glasgewebe zur Verwendung bei der Herstellung von Laminaten und als Comonomere bei der Herstellung von Silikon-modifizierten Polyetherpolymeren brauchbar sind. Diese Verbindungen enthalten jedoch keine Schwefel- oder Stickstoffatome.

Die GB-A-1 058 385 offenbart Organosiliciumpolymerzusammensetzungen, die silyl-modifizierte Polyether umfassen, welche als Siegelungsmittel, Kautschuke und als Beschichtungen brauchbar sind. Es wird lediglich eine Oxetanverbindung offenbart (siehe Seite 12, Beispiel 4). Diese Verbindung enthält ebenfalls weder ein Schwefel- noch ein Stickstoffatom.

Die US-A-5 463 084 offenbart photohärtbare Siliconoxetane, die in Schutzbeschichtungen, dekorativen und isolierenden Beschichtungen, Drucktinten, Siegelmitteln, Klebstoffen , Photowiderständen, Kabelisolierungen usw. brauchbar sind. Die offenbarten offenbarten difunktionellen Oxetanmonomere enthalten wiederum keinerlei Schwefelatom oder Stickstoffatom.

Der Erfindung liegt nunmehr die Aufgabe zugrunde, hydrolysierbare und polymerisierbare Oxetansilane zur Verfügung zu stellen, aus denen allein oder zusammen mit anderen hydrolytisch kondensierbaren und polymerisierbaren Komponenten stabile Zusammensetzungen herstellbar sind, die nur unter geringem Schrumpf und hoher Geschwindigkeit bei Raumtemperatur polymerisieren und als Verbund- oder Beschichtungsmaterial, Klebstoff oder Haftvermittler oder zur Herstellung von Füllstoffen oder Materialien für medizinische oder dentale Zwecke geeignet sind. Diese Silane sollen sich in organisch-anorganische Verbundmaterialien kovalent einbauen lassen und synthetisch so zugänglich sein, daß der Abstand zwischen Silicium und den polymerisierbaren Gruppen variiert werden kann.

Diese Aufgabe wird erfindungsgemäß durch die hydrolysierbaren und polymerisierbaren Oxetansilane nach den Ansprüchen 1 bis 4 gelöst. Die Erfindung betrifft weiter die Kieselsäurekondensate nach Anspruch 5, die Polymerisate nach Anspruch 6, die Zusammensetzungen nach den Ansprüchen 7 und 8 sowie die Verwendung nach Anspruch 9.

Die erfindungsgemäßen hydrolysierbaren und polymerisierbaren Oxetansilane und deren Stereoisomere entsprechen der allgemeinen Formel (I): 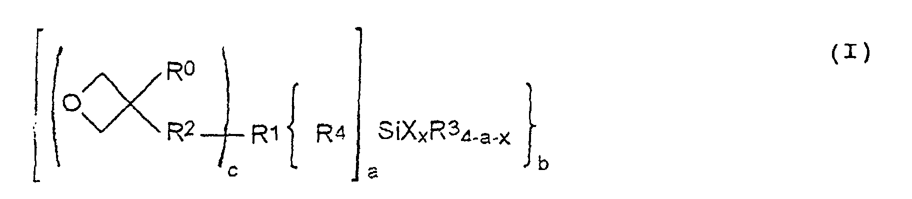 wobei die Variablen R⁰, R¹, R², R³, R⁴, R⁵, R⁶, X, Y, a, b, c, und x unabhängig voneinander die folgenden Bedeutungen haben:
- R⁰ =: Wasserstoff oder substituiertes oder unsubstituiertes C₁- bis C₁₀-Alkyl;
- R¹ =: entfällt oder substituiertes oder unsubstituiertes C₁- bis C₁₈-Alkylen, C₆- bis C₁₈-Arylen, C₇- bis C₁₈-Alkylenarylen oder -Arylenalkylen, wobei diese Reste durch mindestens eine Gruppe ausgewählt aus Ether-, Thioether-, Ester-, Carbonyl-, Amid- und Urethangruppe unterbrochen sein können;
- R² =: entfällt oder substituiertes oder unsubstituiertes C₁- bis C₁₈-Alkylen, C₆- bis C₁₈-Arylen, C₇- bis C₁₈-Alkylenarylen oder C₇- bis C₁₈- Arylenalkylen, wobei diese Reste durch mindestens eine Gruppe ausgewählt aus Ether-, Thioether-, Ester-, Thioester-, Carbonyl-, Amid- und Urethangruppe unterbrochen sein oder diese endständig tragen können;
- R³ =: entfällt oder substituiertes oder unsubstituiertes C₁- bis C₁₈-Alkyl, C₂- bis C₁₈-Alkenyl, C₆- bis C₁₈-Aryl, C₇- bis C₁₈-Alkylaryl oder C₇- bis C₁₈-Arylalkyl, wobei diese Reste durch mindestens eine Gruppe ausgewählt aus Ether-, Thioether-, Ester-, Carbonyl-, Amid- und Urethangruppe unterbrochen sein können;
- R⁴ =: substituiertes oder unsubstituiertes -CHR⁶-CHR⁶-S-R⁵-, -S-R⁵- oder -Y-CO-NH-R⁵-;
- R⁵ =: substituiertes oder unsubstituiertes C₁- bis C₁₈-Alkylen, C₆- bis C₁₈-Arylen, C₆- bis C₁₈-Alkylenarylen oder C₆- bis C₁₈-Arylenalkylen, wobei diese Reste durch mindestens eine Gruppe ausgewählt aus Ether-, Thioether-, Ester-, Carbonyl-, Amid- und Urethangruppe unterbrochen sein können;
- R⁶ =: Wasserstoff oder substituiertes oder unsubstituiertes C₁- bis C₁₈-Alkyl oder C₆ bis C₁₀-Aryl;
- X =: eine hydrolysierbare Gruppe, nämlich Halogen, Hydroxy, Alkoxy oder Acyloxy;
- Y =: O oder S;
- a =: 1, 2 oder 3;
- b =: 1, 2 oder 3;
- c =: 1 bis 6; und
- x =: 1, 2 oder 3;
und mit der Maßgabe, daß
(i) a+x = 2, 3 oder 4
   und
(ii) a und/oder b = 1.

Die obige Formel decken jedoch nur solche Verbindungen ab, die mit der Valenzlehre zu vereinbaren sind.

Üblicherweise liegen die erfindungsgemäßen Silane als Stereoisomeren-Gemische und insbesondere als Racemate vor.

Die bei den Resten möglicherweise vorhandenen Ether-, Thioether-, Ester-, Thioester-, Carbonyl-, Amid- und Urethangruppen sind durch die folgenden Formeln definiert: -O-, -S-, -CO-O-, -O-CO-, -CO-S-, -S-CO-, -CS-O-, -O-CS-, -CO-, -CO-NH-, -NH-CO-, -O-CO-NHund -NH-CO-O-.

Die in den Formeln (I) möglichen nicht-aromatischen Reste oder nicht-aromatischen Teile der Reste können geradkettig, verzweigt oder cyclisch sein.

Alkylreste haben bevorzugt 1 bis 8 und besonders bevorzugt 1 bis 4 Kohlenstoffatome. Spezielle Beispiele für mögliche Alkylreste sind Methyl, Ethyl, n- und iso-Propyl, sec.- und tert.-Butyl, n-Pentyl, Cyclohexyl, 2-Ethylhexyl und Octadecyl.

Alkenylreste haben bevorzugt 2 bis 10 und besonders bevorzugt 2 bis 6 Kohlenstoffatome. Spezielle Beispiele für mögliche Alkenylreste sind Vinyl, Allyl- und iso-Butenyl.

Bevorzugte Beispiel für mögliche Aryl-Reste sind Phenyl, Biphenyl und Naphthyl.

Alkoxyreste haben bevorzugt 1 bis 6 Kohlenstoffatome. Spezielle Beispiel für mögliche Alkoxyreste sind Methoxy, Ethoxy, n-Propoxy, iso-Propoxy und tert.-Butoxy.

Acyloxyreste haben vorzugsweise 2 bis 5 Kohlenstoffatome. Spezielle Beispiele sind Acetyloxy und Propionyloxy.

Bevorzugte Alkylenreste leiten sich von den obigen bevorzugten Alkylresten ab und bevorzugte Arylenreste leiten sich von den obigen bevorzugten Arylresten ab.

Bevorzugte aus einer Kombination von nicht-aromatischem und aromatischem Teil bestehende Reste, wie Alkylaryl-, Arylalkyl-, Alkylenarylen- und Arylenalkylenreste, leiten sich von den obigen bevorzugten Alkyl- und Arylresten ab. Spezielle Beispiele hierfür sind Benzyl, 2-Phenylethyl und Tolyl.

Die genannten substituierten R-Reste tragen einen oder mehrere einfache Substituenten. Beispiele für diese Substituenten sind Methyl, Ethyl, Phenyl, Benzyl, Hydroxymethyl, Hydroxyethyl, Methoxy, Ethoxy, Chlor, Brom, Hydroxy, Mercapto, Isocyanato, Vinyloxy-, Acryloxy-, Methacryloxy-, Allyl-, Styryl, Epoxy, Carboxyl, SO₃H, PO₃H₂ oder PO₄H₂.

Für a, b, c oder x ≥ 2 können die Reste X sowie die einzelnen R-Reste jeweils dieselbe oder eine unterschiedliche Bedeutung haben.

Außerdem existieren für die oben angegebenen Variablen der Formel (I) bevorzugte Definitionen, die, unabhängig voneinander gewählt werden können und wie folgt sind:
- R⁰ =: Wasserstoff oder C₁- bis C₅-Alkyl;
- R¹ =: C₁- bis C₈-Alkylen, wobei diese Reste durch mindestens eine Gruppe ausgewählt aus Ether-, Thioether-, Ester- und Urethangruppe unterbrochen sein können;
- R² =: entfällt oder C₁- bis C₈-Alkylen, wobei diese Reste durch mindestens eine Gruppe ausgewählt aus Ether-, Thioether-, Ester-, Thioester, Carbonyl-, Amid- und Urethangruppe unterbrochen sein oder diese endständig tragen können;
- R³ =: entfällt oder Methyl, Ethyl oder Phenyl;
- R⁴ =: -S-R⁵- oder -Y-CO-NH-R⁵-;
- R⁵ =: C₁- bis C₈-Alkylen, wobei diese Reste durch mindestens eine Gruppe ausgewählt aus Ether-, Thioether-, Ester-, Carbonyl-, Amid- und Urethangruppe unterbrochen sein können;
- R⁶ =: Wasserstoff oder C₁- bis C₅-Alkyl;
- X =: Methoxy, Ethoxy oder Chlor;
- Y =: O oder S;
- a =: 1;
- b =: 1;
- c =: 1 bis 6;
- x =: 2 oder 3; und/oder
- a+x =: 3.

Dabei können die einzelnen R-Reste wiederum einfache Substituenten tragen.

Bevorzugte Verbindungen sind demgemäß solche, bei denen mindestens eine der Variablen der Formel (I) die vorstehend beschriebene bevorzugte Definition aufweist.

Weiter sind solche Oxetansilane der Formel (I) bevorzugt, bei denen die Indices a, b und/oder c den Wert 1 haben, und Beispiele hierfür sind die Silane gemäß den nachstehenden allgemeinen Formeln (II), (III), (IV) und (V).

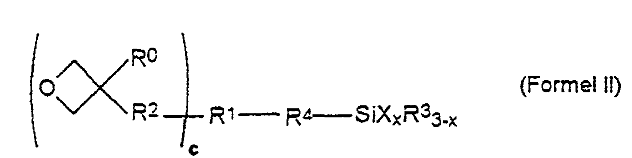

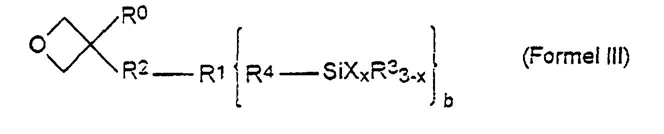

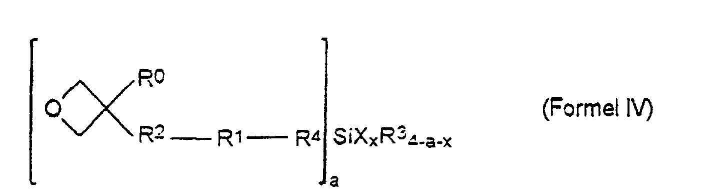

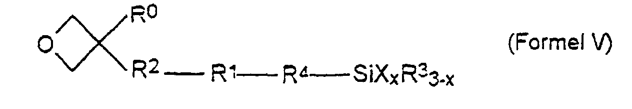

Spezielle Beispiele für bevorzugte erfindungsgemäße Oxetansilane der Formel (I) sind im folgenden angegeben:

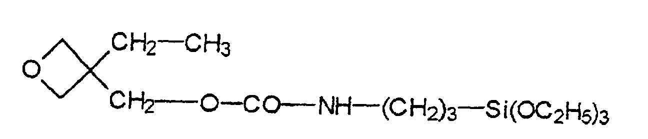

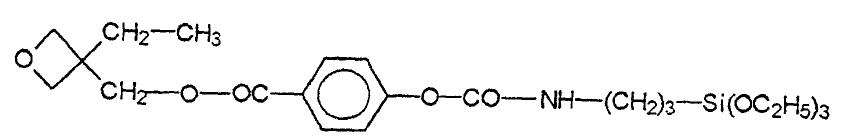

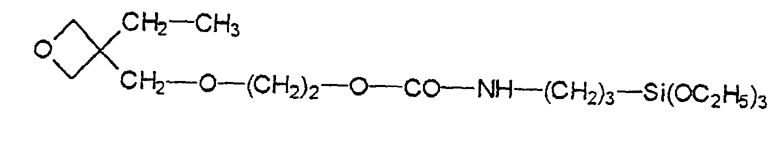

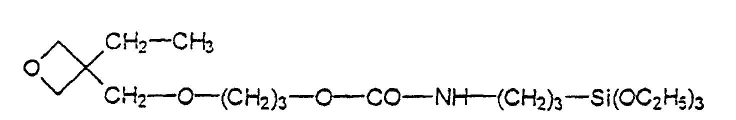

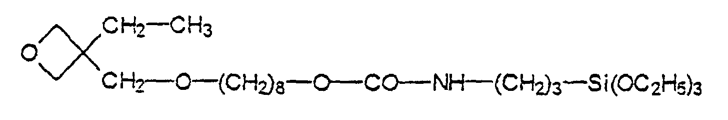

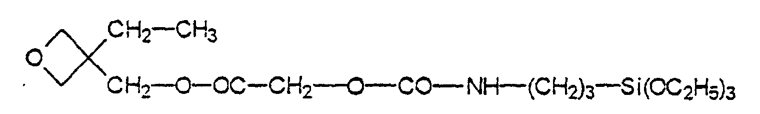

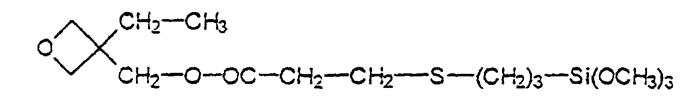

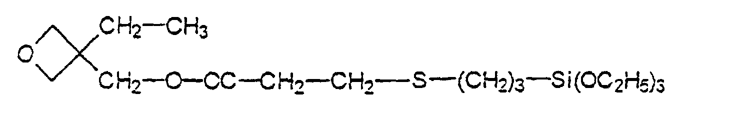

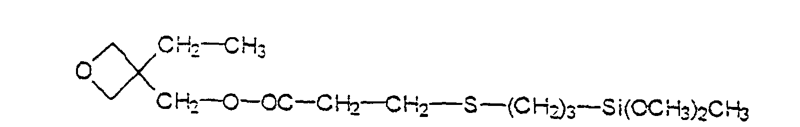

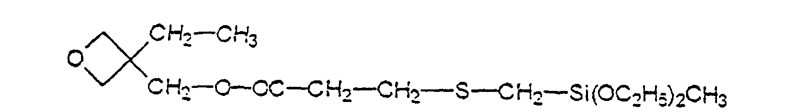

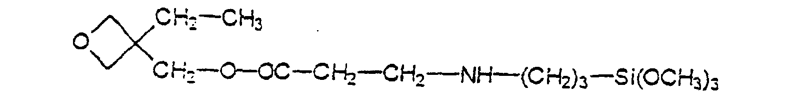

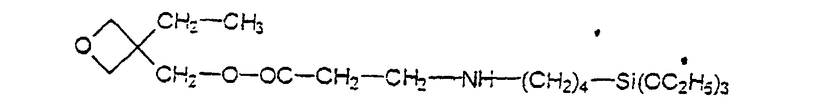

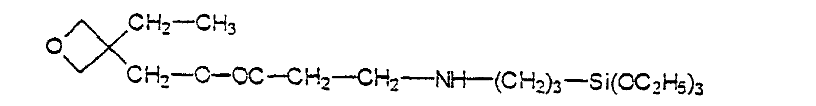

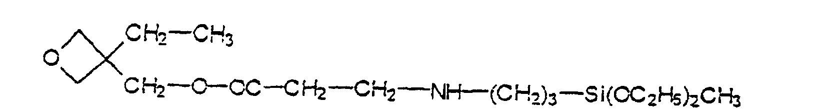

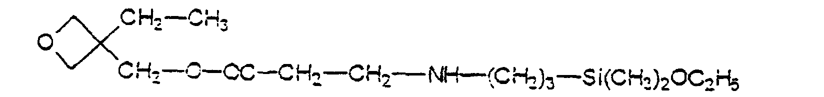

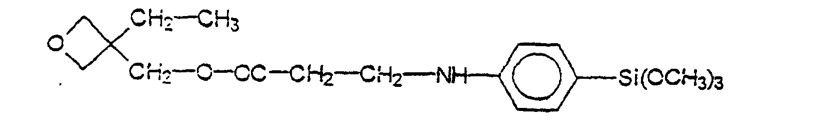

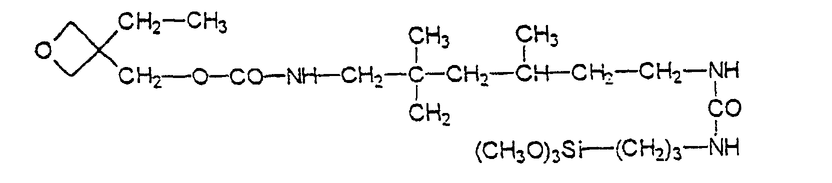

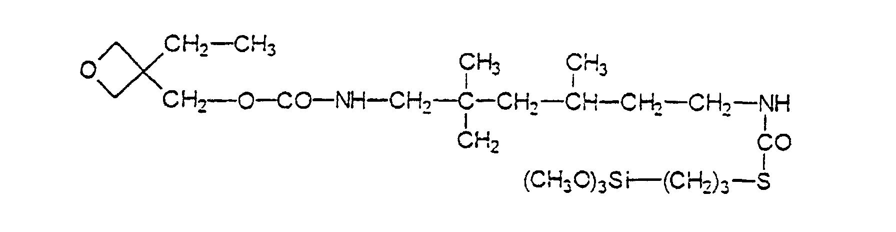

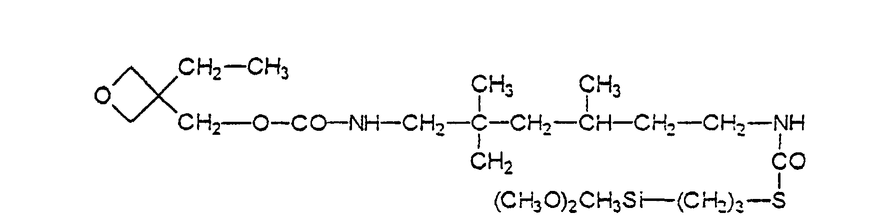

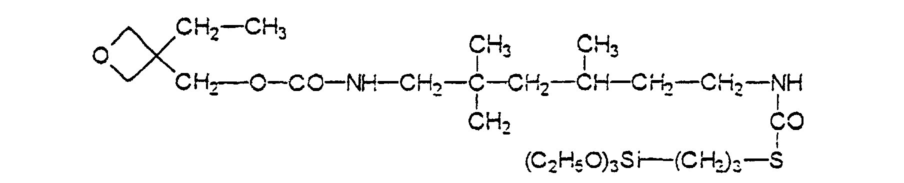

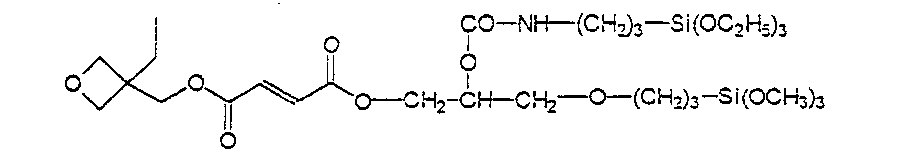

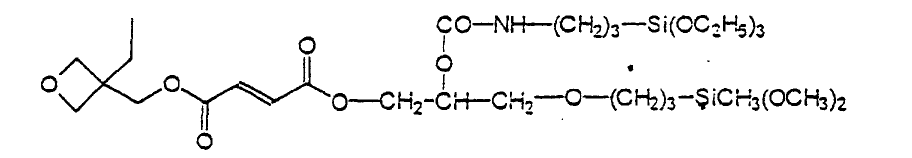

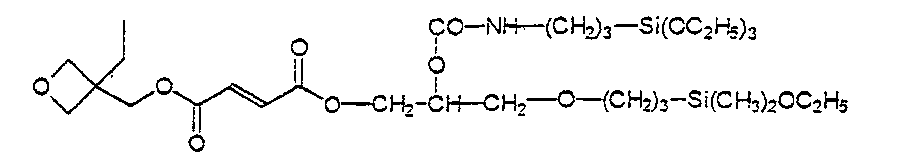

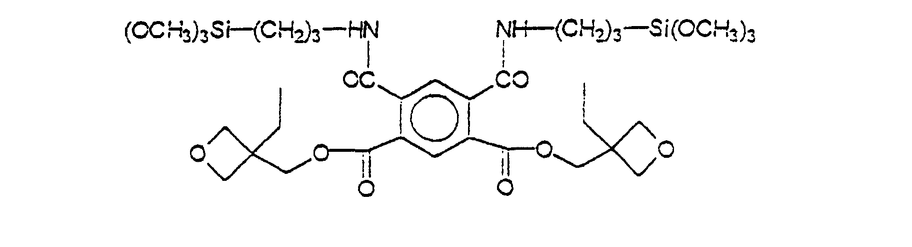

Die Herstellung der erfindungsgemäßen Oxetansilane (I) ist insbesondere über eine große Anzahl von konventionellen Additions- oder Kondensationsreaktionen möglich, die nach den für diese Reaktionen üblichen Methoden durchgeführt werden. Verfahren die zur Herstellung der erfindungsgemäßen Silane eingesetzt werden können, sind z.B. in W. Noll, Chemie und Technologie der Silicone, 2. Auflage, Verlag Chemie, Weinheim 1968, insbesondere S. 22 ff., sowie in dem Übersichtsartikel von R.C. Mehrotra in J. Non-Crystalline Solids 100, (1988) 1-15 und der in diesem Artikel zitierten Literatur beschrieben.

In einer ersten Variante kann z.B. 3-Ethyl-3-hydroxymethyloxetan (1) an ein isocyanatgruppenhaltiges Silan addiert werden:

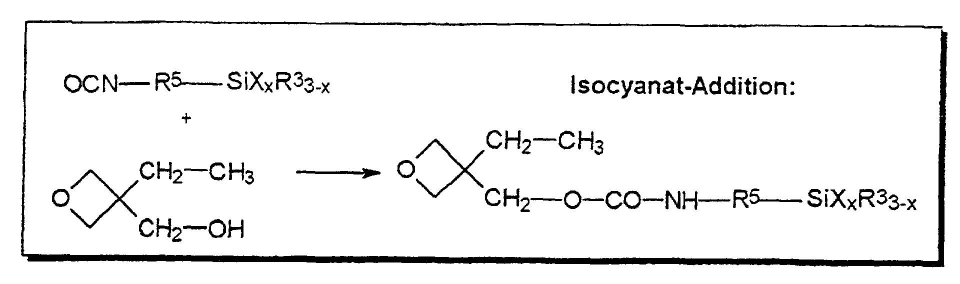

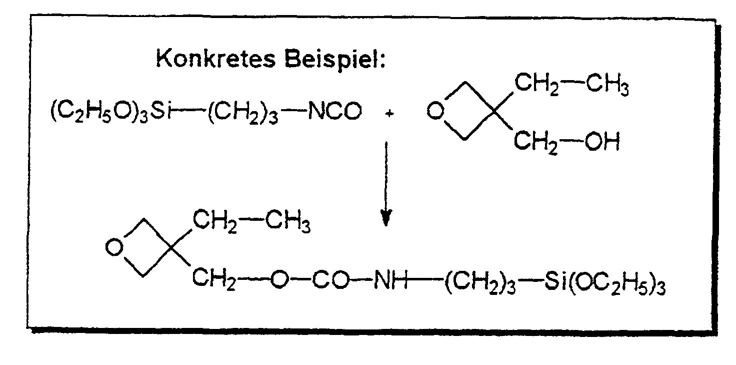

Weiter ist ausgehend von 3-Acryloyloxymethyl-3-ethyloxetan (2) z.B. die Thiol-En-Addition mit Mercaptosilanen möglich:

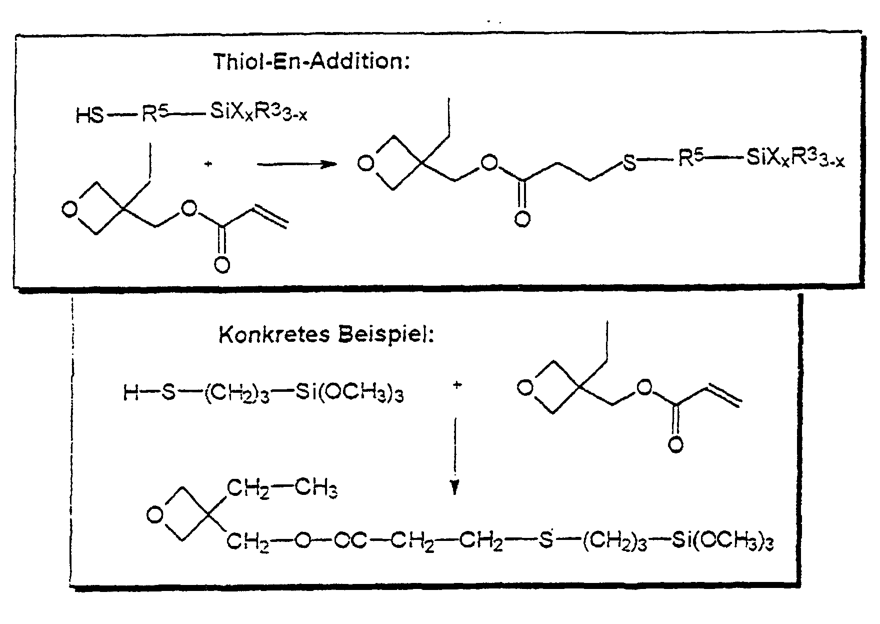

Darüber hinaus kann der Halbester aus Fumarsäure und 3-Ethyl-3-hydroxymethyl-oxetan an ein Epoxidsilan addiert werden:

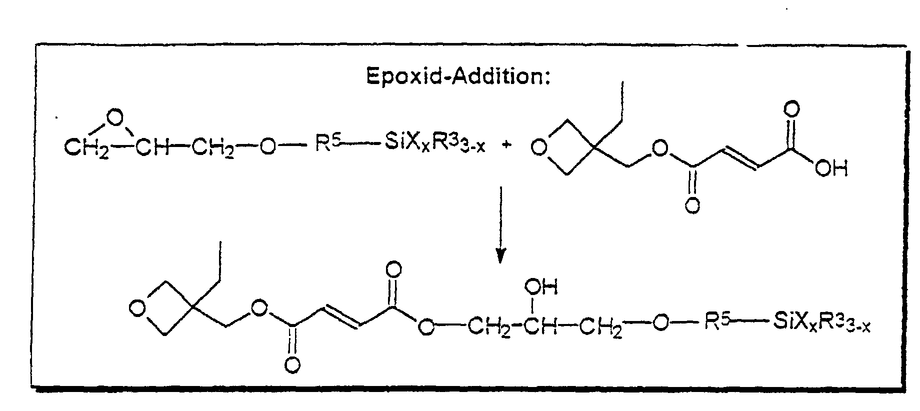

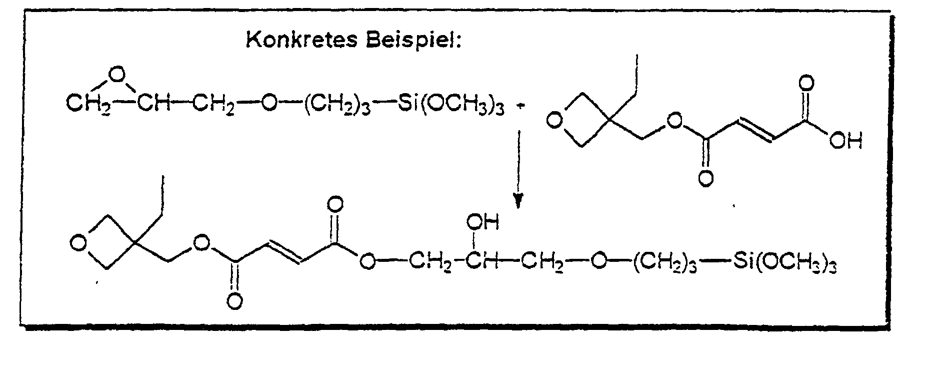

Das erhaltene Silan läßt sich mit einem isocyanatgruppenhaltigen Silan weiter umsetzen, so daß Oxetansilane mit zwei Silylgruppen erhalten werden:

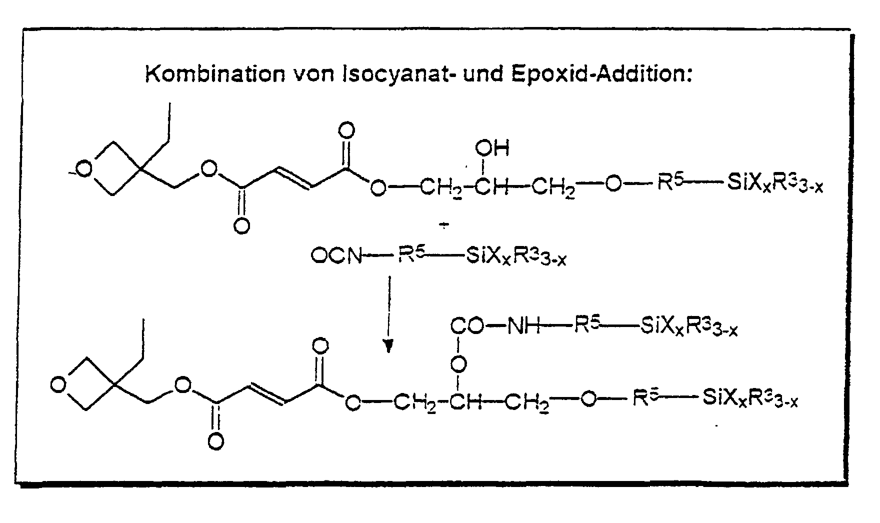

Silane mit mehreren Oxetan-Resten sind über Additionsreaktionen zugänglich. So kann z.B. durch Reaktion von (1) mit Tetracarbonsäuredianhydriden, wie Pyromellithsäureanhydrid, 1,2,3,4-Butantetracarbonsäuredianhydrid oder Tetrahydrofuran-2,3,4,5-tetracarbonsäuredianhydrid, ein Addukt erhalten werden, welches weiter mit 1 oder 2 Mol eines Epoxid- oder Isocyanatosilan umgesetzt wird.

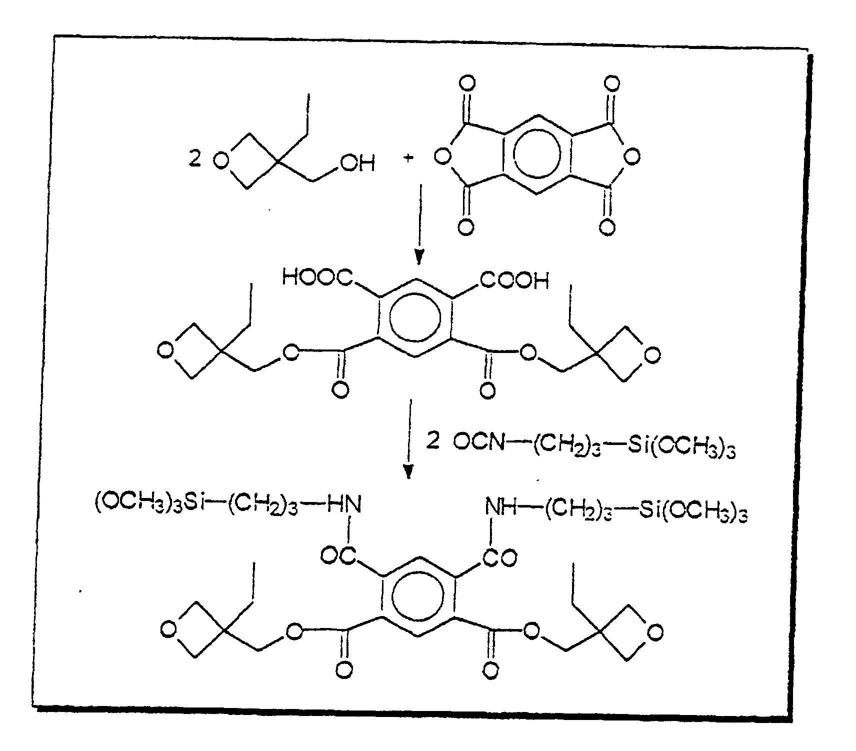

Die erfindungsgemäßen Silane (I) sind über die Oxetan-Gruppen polymerisierbar und über die Reste X hydrolysierbar. Dabei führt die Polymerisation der Oxetangruppen zum Aufbau eines organischen Netzwerkes, während die hydrolysierbaren Gruppen durch Polykondensation ein anorganisches Polysiloxan-Netzwerk ergeben.

Die erfindungsgemäßen Oxetansilane stellen Stoffe hoher Reaktivität dar, die bei Hydrolyse polymerisierbare Kieselsäurekondensate bilden, welche in Gegenwart von üblichen kationischen Initiatoren oder Photoinitiatoren bei Raumtemperatur oder bei Einstrahlung von Licht des sichtbaren oder UV-Bereichs zu mechanisch stabilen Schichten, Form- oder Füllkörpern polymerisiert werden können.

Durch geeignete Auswahl der bei der Herstellung der Oxetansilane eingesetzten Edukte kann die Anzahl der hydrolysierbaren Gruppen, der polymerisierbaren Gruppen und weiterer funktioneller Gruppen variiert werden. In Abhängigkeit von der Art und Anzahl der hydrolysierbaren Gruppen, z.B. Alkoxygruppen und der Anzahl der Oxetangruppen führt die Kondensation der Oxetansilane und die Polymerisation der erhaltenen Kondensate daher zu Materialien mit Eigenschaften, die von silicongummiartig bis glasartig reichen. Im Vergleich zu radikalisch polymerisierbaren Silanen tritt bei der Polymerisation der erfindungsgemäßen Oxetan-Silane keine Inhibierungsschicht auf, was gerade bei der Herstellung von Beschichtungen sehr vorteilhaft ist.

Bei Anwesenheit von mindestens zwei Oxetan-Resten ist die Ausbildung eines dreidimensionalen, organischen Netzwerkes möglich, wobei über den Abstand zwischen dem Si-Atom und dem Oxetan-Rest, d.h. über die Länge der Spacergruppe, und durch Einbau weiterer funktioneller Gruppen die mechanischen Eigenschaften, wie z.B. Festigkeit und Flexibilität, und die physikalisch-chemischen Eigenschaften, z.B. Haftungsvermögen, Wasseraufnahme und Brechzahl, der erhaltenen Kieselsäurekondensate variiert und den Anforderungen des jeweiligen Anwendungsfalles optimal angepaßt werden können. Dabei führen aliphatische Gruppen zu eher flexiblen und aromatische Gruppen zu eher steifen Produkten.

Durch die Anzahl der polymerisierbaren Oxetangruppen ist ferner die Vernetzungsdichte einstellbar, die dann ebenfalls die Eigenschaften und Einsatzmöglichkeiten der entsprechenden Kieselsäurekondensate beeinflußt. Enthalten die erfindungsgemäßen Oxetansilane darüber hinaus noch ionisch vernetzbare Gruppen, wie z.B. (Meth)acrylat, Styryl oder Allyl, dann kann simultan oder konsekutiv, d.h. als 2-Stufen-Prozeß, durch deren radikalische Polymerisation eine weitere Erhöhung der Vernetzungsdichte erreicht werden.

Die erfindungsgemäßen Oxetansilane und deren Rieselsäurekondensate besitzen eine nur geringe Flüchtigkeit, so daß sie sich einfach und weitgehend unbedenklich verarbeiten lassen. Im Hinblick auf die vorstehend angegebenen Variationsmöglichkeiten der kondensierbaren und polymerisierbaren Reste der erfindungsgemäßen Oxetansilane können daraus herstellbare Kieselsäurekondensate als Harze oder Füllstoffe für verschiedenste Anwendungsgebiete bereitgestellt werden können.

Die Silane (I) sind stabile Verbindungen, sie können entweder allein oder zusammen mit anderen hydrolysierbaren, kondensierbaren und/oder polymerisierbaren Komponenten zu den erfindungsgemäßen Kieselsäurekondensaten verarbeitet werden.

Neben den Silanen der Formel (I) können noch weitere hydrolytisch kondensierbare Verbindungen des Siliciums, Aluminiums, Titans, Zirkoniums oder Phosphors bei der Herstellung der erfindungsgemäßen Kieselsäurekondensate eingesetzt werden, die dann auch als Kieselsäure(hetero)kondensate bezeichnet werden. Diese Verbindungen können entweder als solche oder in bereits vorkondensierter Form verwendet werden. Bevorzugt ist es, wenn für die Herstellung der erfindungsgemäßen Kieselsäure(hetero)kondensate mindestens 20 Mol.%, besonders bevorzugt mindestens 80 Mol.%, auf der Basis monomerer Verbindungen, hydrolysierbare Siliciumverbindungen eingesetzt werden. Ebenso bevorzugt ist es, wenn zu Herstellung der Kieselsäure(hetero)kondensate mindestens 10 Mol.%, insbesondere 40 bis 100 Mol.%, jeweils auf der Basis monomerer Verbindungen, erfindungsgemäße Oxetansilane verwendet werden.

Als weitere hydrolytisch kondensierbare Verbindungen wird bevorzugt mindestens ein Silan der allgemeinen Formel (VI) eingesetzt:

R⁷ ₖ(Z'R⁸)ₘSiX'₄₋₍ₖ₊ₘ₎ (VI)

wobei R⁷, Z', R⁸, X', k und m, sofern nicht anders angegeben, unabhängig voneinander die folgenden Bedeutungen haben:
- R⁷ =: C₁- bis C₈-Alkyl, C₂- bis C₁₂-Alkenyl oder C₆- bis C₁₄-Aryl;
- R⁸ =: C₁- bis C₈-Alkylen, C₂- bis C₁₂-Alkenylen oder C₆- bis C₁₄-Arylen;
- X' =: Wasserstoff, Halogen oder C₁- bis C₈-Alkoxy;
- Z' =: Mercapto-, Glycidyl-, Acryl-, Methacryl-, Vinyl-, Allyl- oder Vinylethergruppe;
- k =: 0, 1, 2 oder 3;
- m =: 0, 1, 2 oder 3; und
- k+m =: 1, 2 oder 3.

Derartige Silane sind z.B. in der DE-C-34 07 087 beschrieben, und spezielle Beispiele für hydrolytisch kondensierbare Silane der allgemeinen Formel (VI) sind: CH₃-Si-Cl₃, CH₃-Si-(OC₂H₅)₃, C₂H₅-Si-Cl₃, C₂H₅-Si-(OC₂H₅)₃, CH₂=CH-Si-(OC₂H₅)₃, CH₂=CH-Si-(OCH₃)₃, CH₂=CH-Si-(OC₂H₄OCH₃)₃, (CH₃)₂-Si-Cl₂, (CH₃)₂-Si-(OC₂H₅)₂, (C₂H₅)₃-Si-Cl, (C₂H₅)₂-Si-(OC₂H₅)₂, (CH₃)₃-Si-Cl, (CH₃O)₃-Si-C₃H₆-NH₂, (CH₃O)₃-Si-C₃H₆-SH, (CH₃O)₃-Si-C₃H₆-NH₂,

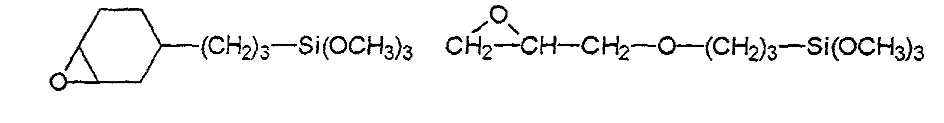

Darüber hinaus können als weitere bevorzugte hydrolytisch kondensierbare Verbindungen mindestens eine Zirkonium-, Titan- oder Aluminium-Verbindung der Formeln

MeX"R⁹ _{z} AlR¹⁰ ₃

eingesetzt werden, wobei Me, R⁹, R¹⁰, X", y und z unabhängig voneinander die folgenden Bedeutungen haben:
- Me =: Zr oder Ti;
- R⁹ =: Wasserstoff, substituiertes oder unsubstituiertes C₁- bis C₁₂-Alkyl, C₇- bis C₁₅-Alkylaryl oder C₆- bis C₁₄-Aryl;
- R¹⁰ =: Halogen, OH, C₁- bis C₈-Alkoxy;
- X" =: Halogen, OH, C₁- bis C₈-Alkoxy;
- y =: 1 bis 4, insbesondere 2 bis 4;
- z =: 1 bis 3, insbesondere 0 bis 2.

Bevorzugte Beispiele für einsetzbare Zirkonium- und Titan- Verbindungen sind ZrCl₄, Zr(OC₂H₅)₄, Zr(OC₃H₇)₄, Zr(OC₄H₉)₄, ZrOCl₂, TiCl₄, Ti(OC₂H₅)₄, Ti(OC₃H₇)₄ und Ti(OC₄H₉)₄. Bevorzugte Beispiele für einsetzbare Aluminium-Verbindungen sind Al(OCH₃)₃, Al(OC₂H₅)₃, Al(OC₃H₇)₃, Al(OC₄H₉)₃ and AlCl₃.

Auch komplexierte Zr-, Ti- und Al-Verbindungen können eingesetzt werden, wobei als Komplexbildner u.a. Säuren oder β-Dicarbonylverbindungen fungieren können.

Weitere hydrolysierbare Verbindungen, die zur Herstellung der Kieselsäure(hetero)kondensate eingesetzt werden können, sind z.B. Bortrihalogenide, Zinntetrahalogenide, Zinntetraalkoxide und Vanadylverbindungen.

Die erfindungsgemäßen Kieselsäurekondensate der Silane (I) werden durch Hydrolyse der vorhandenen hydrolysierbaren Gruppen X, z.B. Alkoxy-Gruppen, und anschließende Kondensation erhalten, welche zur Ausbildung eines anorganischen Netzwerkes aus Si-O-Si-Einheiten führt. Die Hydrolyse und Kondensation erfolgt üblicherweise im basischen oder sauren Milieu, wobei eine Verknüpfung von C=C-Doppelbindungen, die in den eingesetzten Silanen enthalten sind, in der Regel unerwünscht ist.

Die erfindungsgemäßen Kieselsäurekondensate können auch in nicht vollständig hydrolysierter und kondensierter Form vorliegen. In solchen Fällen spricht man auch von sogenannten Vorkondensaten.

Bei der Herstellung der erfindungsgemäßen Kieselsäure(hetero)kondensate geht man üblicherweise so vor, daß man die gegebenenfalls in einem Lösungsmittel gelösten Silane (I) bei Raumtemperatur oder unter leichter Kühlung und in Gegenwart eines Hydrolyse- und Kondensationskatalysators mit der erforderlichen Menge Wasser versetzt und die entstehende Mischung ein bis mehrere Stunden lang rührt. Als Lösungsmittel kommen vor allem aliphatische Alkohole, wie z.B. Ethanol oder i-Propanol, Dialkylketone, wie Aceton oder Methylisobutylketon, Ether, wie z.B. Diethylether oder Tetrahydrofuran (THF), Ester, wie Ethyl- oder Butylacetat, und deren Mischungen in Frage.

Wird die hydrolytische Kondensation in Gegenwart reaktiver Zr-, Ti- oder Al-Verbindungen durchgeführt, so sollte die Wasserzugabe stufenweise bei ca. 0 bis 30 °C erfolgen. Dabei ist es meist günstig, Wasser nicht als solches zuzugeben, sondern in Form von wasserhaltigen Lösungsmitteln, wie z.B. wäßrigem Ethanol, oder durch Freisetzung über eine chemische Reaktion, wie z.B. über eine Veresterung, zuzuführen.

Vorzugsweise erfolgt die Hydrolyse und Kondensation in Gegenwart eines Kondensationskatalysators, wobei protonen- oder hydroxylionen-abspaltende Verbindungen, wie organische oder anorganische Säuren oder Basen, bevorzugt sind. Besonders bevorzugt sind flüchtige Säuren oder Basen, insbesondere Salzsäure oder Ammoniak. Es hat sich bewährt, bei der Hydrolyse und Kondensation Verfahrensweisen der Sol-Gel-Technologie zu übernehmen, wie sie z.B. in C.J. Brinker et al., "Sol-Gel-Science", Academic Press, Boston, 1990, beschrieben sind. Das "Sol-Gel-Verfahren" ist darüber hinaus in DE-A-27 58 414, DE-A-27 58 415, DE-A-30 11 761, DE-A-38 26 715 und DE-A-38 35 968 offenbart.

Die erhaltenen Kieselsäure(hetero)kondensate der Silane (I) und gegebenenfalls weiterer hydrolytisch kondensierbare Verbindungen können entweder als solche oder nach teilweiser oder vollständiger Entfernung von verwendetem Lösungsmittel eingesetzt werden. In einigen Fällen kann es sich auch als vorteilhaft erweisen, das zur hydrolytischen Kondensation eingesetzte Lösungsmittel durch ein anderes Lösungsmittel zu ersetzen.

Die erfindungsgemäßen polymerisierbaren Kieselsäure(hetero)kondensate und die Silane (I) sowie Zusammensetzungen mit Gehalt an diesen Kondensaten oder Silanen können durch kationische Polymerisation oder Photopolymerisation gehärtet werden, wobei die Polymerisation üblicherweise nach Zugabe geeigneter Initiatoren und weiterer polymerisationsfähiger Komponenten erfolgt. Wenn unterschiedliche polymerisationsfähige Gruppen, z.B. Oxetan- und (Meth)acrylgruppen, vorhanden sind, können auch mehrere Härtungsmechnismen, z.B. kationische und radikalische Polymerisation, gleichzeitig oder in aufeinander folgenden Stufen benutzt werden.

Zur Initiierung der kationischen Polymerisation werden vorzugsweise kationische Initiatoren und/oder Photoinitiatoren eingesetzt.

Bevorzugte Beispiele für kationische Initiatoren sind starke Brönstedt- und Lewis-Säuren, z.B. Schwefelsäure, Trifluoressigsäure, Aluminiumtrichlorid oder Bortrifluorid.

Geeignete Photoinitiatoren sind Oniumsalze, Triarysulfoniumsalze, Diaryliodoniumsalze, Cyclopentadienyleisen(I)-salze und Isochinolinsalze. Besonders geeignet sind eine Mischung aus 4-(Diphenylsulfino)-Phenylphenylsulfid-bis-hexafluoroantimonat und Bis[4-(diphenylsulfino)-phenyl]sulfid-hexafluoroantimonat (Cyraure UVI 6974, Union Carbide), Bis[4-(diphenylsulfino)-phenyl]sulfidhexafluorophosphat (Degacure Kl-85, Degussa), Diphenyliodoniumhexafluoroantimonat oder -hexafluorophosphat und der (η⁵-2,4-Cyclopentadien-1-yl)[1,2,3,4,5,6-η]-(cumol)-eisen(1)-hexafluorophosphin-Komplex (Irgacure 261, Ciba-Geigy). Durch Sensibilisatoren, wie Thioxanthon-Derivate, Campherchinon, Phenanthrenchinon oder Perylen, kann die Empfindlichkeit im sichtbaren Bereich erhöht werden. Weiterhin erweist es sich auch als günstig, die Photopolymerisation in Gegenwart radikalischer Photoinitiatoren, wie z.B. Benzoinalkylethern, Benzildialkylketalen oder Acrylphosphinoxiden, durchzuführen.

In den erfindungsgemäßen Zusammensetzungen können neben den Silanen (I) oder den entsprechenden Kieselsäure(hetero)kondensaten auch geeignete polymerisationsfähige mono- oder multifunktionelle Monomere vorhanden sein, welche auch als Verdünnermonomere bezeichnet werden können.

Besonders geeignete Verdünnermonomere sind oxetangruppenhaltige Monomere, wie z.B. 3-Ethyl-3-hydroxymethyloxetan (1), 3,7-Bis(3-oxetanyl)-5-oxa-nonan (3), 3,3'-(1,2-Ethandiylbis(oxymethylen))-bis(3-ethyloxetane) (4), 3,3'-(1,10-Decandiylbis(oxymethylen))-bis(3-ethyloxetane) (5), 3,3'-(1,3-(2-Methylenyl)propandiylbis(oxymethylen))-bis(3-ethyloxetane) (6) oder 3,3'-(1,4-Xylendiylbis(oxymethylen))-bis(3-ethyloxetane) (7):

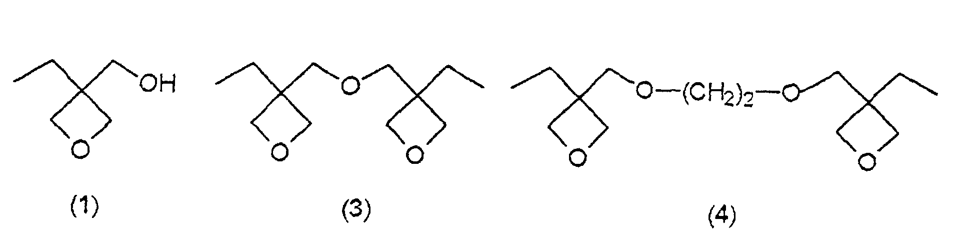

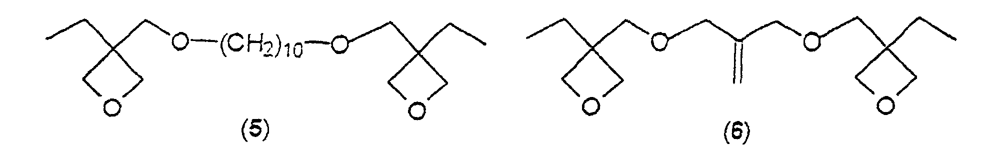

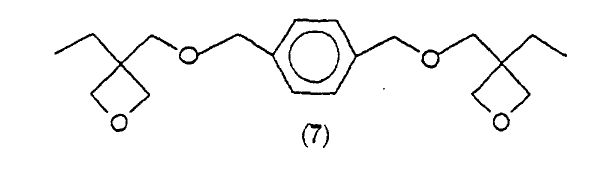

Diese Verbindungen sind bekannt (vgl. H. Sasaki, J.V. Crivello, J. Macromol. Sci.-Pure Appl. Chem. **A29** (1992) 915-930; J.V. Crivello, H. Sasaki, J. Macromol. Sci.-Pure Appl. Chem. **A30** (1993) 189-206).

Darüber hinaus sind auch radikalisch polymerisierbare Verdünnermonomere wie mono-funktionelle (Meth)acrylate, z.B. Methyl(meth)acrylat, Ethyl(meth)acrylat, Butyl(meth)acrylat, Benzyl(meth)acrylat, Furfuryl(meth)acrylat oder Phenyl(meth)acrylat, sowie mehrfunktionelle (Meth)acrylate, z.B. Bisphenyl-A-di(meth)-acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat oder 1,12-Dodecandioldi(meth)acrylat, einsetzbar.

Die erfindungsgemäßen Silane, deren Kieselsäurekondensate oder Kieselsäureheterokondensate sowie diese enthaltende Zusammensetzungen können als solche oder in zumindest teilweise polymerisierter Form als Lacke zur Beschichtung von Kunststoffen, Glas oder anderen Substraten, als Füllstoffe oder Bulkmaterial für Komposite und zur Herstellung von medizinischen Materialien, wie Kontaktlinsen, eingesetzt werden. Besonders bevorzugt werden sie jedoch als Dentalmaterial oder Bestandteil davon verwendet.

Gegebenenfalls können die erfindungsgemäßen Zusammensetzungen auch weitere Additive, wie z.B. Färbemittel (Pigmente oder Farbstoffe), Stabilisatoren, Aromastoffe, mikrobizide Wirkstoffe, Flammschutzmittel, Weichmacher oder UV-Absorber, enthalten.

Weitere bevorzugte Additive sind Füllstoffe. Beispiele für bevorzugte Füllstoffe sind Quarz-, Glaskeramik- oder Glaspulver, insbesondere Barium- oder Strontiumsilikatglas-Pulver, Lithium-Aluminium-Silikatglas-Pulver, Silicium-, Zirkonium- oder Aluminiumoxid, oder deren Mischungen, feinteilige Kieselsäuren, insbesondere pyrogene oder gefällte Kieselsäuren, und röntgenopake Füllstoffe, wie z.B. Ytterbiumtrifluorid.

Eine besonders bevorzugte erfindungsgemäße Zusammensetzung enthält:
(a) 5 bis 90, insbesondere 10 bis 70 Gew.-%, bezogen auf die Zusammensetzung, Kieselsäure(hetero)kondensat von einem Silan (I),
(b) 0 bis 80, insbesondere 0 bis 50 Gew.-%, bezogen auf die Zusammensetzung, Verdünnermonomer,
(c) 0,1 bis 5, insbesondere 0,2 bis 2,0 Gew.-%, bezogen auf die Zusammensetzung, Polymerisationsinitiator, und/oder
(d) 0 bis 90, insbesondere 0 bis 80 Gew.-%, bezogen auf die Zusammensetzung, Füllstoffe.

Besonders bevorzugt werden die erfindungsgemäßen Zusammensetzungen als dentaler Zement, dentales Füllungsmaterial oder dentales Bonding für Füllungsmaterialien eingesetzt. Die Verwendung der Zusammensetzungen erfolgt dabei insbesondere dadurch, daß sie auf den zu behandelnden Bereich eines künstlichen oder natürlichen Zahnes aufgebracht und durch Polymerisation gehärtet werden.

Dabei erweist es sich als besonderer Vorteil der erfindungsgemäßen Zusammensetzungen, daß diese einerseits einen nur geringen Polymerisationsschrumpf zeigen und andererseits zu Kompositmaterialien mit hoher mechanischer Festigkeit führen. Eine derartige Kombination von Eigenschaften ist gerade bei Dentalmaterialien von besonderer Bedeutung.

Die Erfindung wird im folgenden anhand von Beispielen näher erläutert.

### Beispiel 1: Synthese von 7-Trimethoxysilyl-4 -thia-heptansäure-(3-ethyl-oxetan-3-yl)methylester (8)

### 1. Stufe: 3-Ethyloxetan-3-yl-methylacrylat

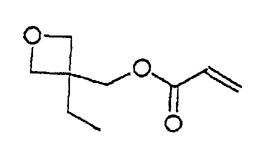

62,3 g (688 mmol) Acrylsäurechlorid in 300 ml Diethylether wurden zu einer eisgekühlten Lösung von 80 g (688 mmol) 3-Ethyl-3-hydroxyethyl-oxetan und 77,3 g Collidin (688 mmol) in 400 ml Diethylether zugetropft. Nach 6 h Rühren bei Raumtemperatur wurde das gebildete Hydrochlorid abfiltriert, und das Filtrat wurde mit wäßriger Salzsäure und mit NaHCO₃-Lösung gewaschen. Nach dem Trocknen mit wasserfreiem Na₂SO₄ und zusätzlichem Stabilisieren mit Hydrochinonmonomethylether wurde das Lösungsmittel am Rotationsverdampfer bei 120 mbar abgezogen. Nach fraktionierter Destillation (Kp._{0,2}:56°C) wurden 57 g (50% Ausbeute) farblose, klare Flüssigkeit erhalten.
- ¹H-NMR:: 5,6-6,6 (m,3H,CH=CH₂), 3,4-4,7 (m,6H,CH₂O), 1,6-1,9 (q,2H,CH₂), 0,7-1,0 (t,3H,CH₃) ppm.
- IR(Film):: 2965, 2874, 1728, 1408, 1268, 1194 cm⁻¹.

### 2. Stufe: 7-Trimethoxysilyl-4 -thia-heptansäure-(3-ethyl-oxetan -3-yl)methylester)

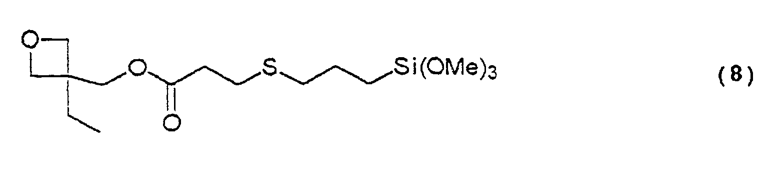

In einer trockenen und mit Argon gespülten Apparatur wurden 17,0 g (0,1 Mol) 3-Ethyl-oxetan-3-yl-methylacrylat zu 19,6 g (0,1 Mol) 3-Mercaptopropyl-trimethoxy-silan gegeben, und es wurde 48 h bei Raumtemperatur gerührt. Nach dem Entfernen aller flüchtigen Bestandteile durch Trocknung bei 60°C bei 0,1 mbar wurden 30 g (81% Ausbeute) einer farblosen Flüssigkeit erhalten.
- ¹H-NMR:: 4,2-4,5 (m,6H,CH₂O), 3,6 (s,9H,CH₃O), 2,3-2,9 (m,6H, CH₂S,CH₂C=O), 1,6-1,0 (m,4H,CH₂), 0,8-1,0 (t,3H,CH₃), 0,6-0,7 (t,2H,CH₂Si) ppm.
- IR (Film):: 2940, 2870, 1737, 1459, 1244, 1089 cm⁻¹.

### Beispiel 2: Synthese von N-(3-Triethoxysilylpropyl)-(3-ethyloxetan-3-yl)-methyl carbamat (9)

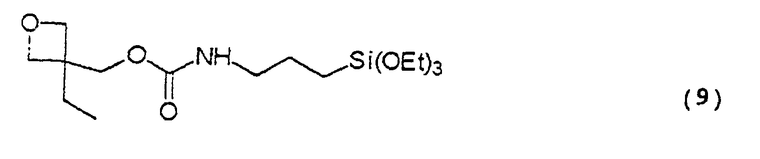

In einer trockenen Apparatur wurden unter Argon 11,6 g (0,1 Mol) 3-(3-Ethyloxetanyl)methanol zu 24,7 g (0,1 Mol) 3-Isocyanatopropyltriethoxysilan und 25 ml wasserfreiem Ether gegeben. Nach Zugabe von 20 mg Dibutylzinndilaureat wurde das Gemisch 6 h bei Raumtemperatur gerührt. Nach Abziehen des Lösungsmittels verblieben 30 g (ca. 85%) einer farblosen Flüssigkeit.

| Elementaranalyse: | | | | |
|---|---|---|---|---|
| C₁₆H₃₃NO₆Si [363,5] | Ber. | C 52,86 | H 9,15 | N 3,85 |
| | Gef. | C 51,52 | H 9,48 | N 3,76 |

- ¹H-NMR:: 5,6 (br,H,NH), 4,4-4,5 (q,4H,CH₂O), 4,2 (s,2H,CH₂O), 3,6-3,8 (q,6HCH₂O), 30,0-3,3 (q,2H,CH₂N), 1,4-1,8 (m,4H,CH₂), 1,1-1,3 (t,9H,CH₃), 0,9 (t,3H,CH₃), 0,4-0,7 (t,2H,CH₂Si) ppm.
- IR (Film):: 3336, 2972, 2930, 2880, 1724, 1533, 1245, 1080 cm¹.

### Beispiel 3: Synthese von 2-(3-Triethoxysilylpropylthio)bernsteinsäure-bis-[(3-ethyloxetan-3-yl)-methyl]-ester (10)

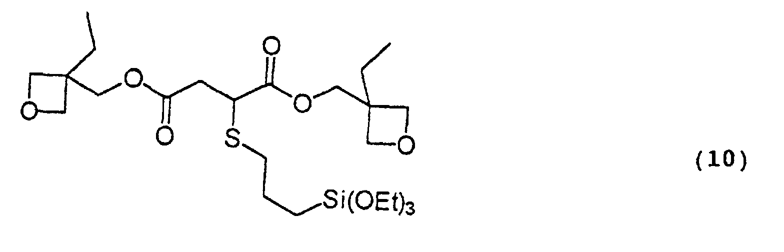

8,4 g (26,9 mmol) Fumarsäure-bis-[(3-ethyloxetan-3-yl)methyl]-ester, 5,3 g (26,9 mmol) 3-Mercaptopropyltriethoxysilan und 0,33 g (1,3 mmol) Dibenzoylperoxid wurden in 20 ml Toluol 5 Stunden lang bei 100°C gerührt. Beim Abdestillieren des Lösungsmittels fiel ein weißer Nederschlag aus, der nach dem Abfiltrieren und Trocknen 10,2 g (70% Ausbeute) an (10) ergab.

### Beispiel 4: Herstellung eines Kieselsäurekondensates auf der Basis von Silan (8)

Es wurden 20 mmol Silan (8) und 20 mmol Dimethyldimethoxysilan in 50 ml wasserfreiem Ethanol gelöst. Nach Zugabe einer Mischung von 50 mmol Wasser und 5 ml Ethanol sowie einiger Tropfen 0,1 molarer ethanolischer Essigsäurelösung wurde 5 Stunden unter Rückfluß erwärmt und man ließ über Nacht Rühren. Nach Entfernen flüchtiger Komponenten im Vakuum konnte das gebildete Harz (7 g) für eine kationische Polymerisation eingesetzt werden.

### Beispiel 5: Herstellung eines dentalen Bondings

Zu einer Mischung aus 4 g Harz aus Beispiel 4 und 3 g 3,7-Bis(3-oxetanyl)-5-oxanonan (3) wurden 70 mg , d.h. 1 Gew.-%, Cyraure UVI 6974 (Union Carbide) gegeben. Anschließend wurde die Mischung als Film ausgegossen und 60 s in einem dentalen Belichtungsgerät, nämlich Heliomat (Firma Vivadent), bestrahlt. Es bildete sich ein fester, gut haftender Film. Von dem Ausgangsharz und dem Polymerisat wurde jeweils die Dichte nach der Auftriebsmethode bestimmt, wobei sich aus der Dichtedifferenz die Volumenänderung, d.h. der Schrumpf, während der kationischen Ringöffnungspolymerisation ergab. Der ermittelte ΔV-Wert von nur -4,1% lag deutlich niedriger als bei konventionalen Bondings auf Methycrylatbasis. So liegt der Volumenschrumpf bei der Härtung von dem kommerziell erhältlichen Bonding Heliobond (Firma Vivadent) bei 7,5%.

## Patentansprüche

1. Hydrolysierbare und polymerisierbare Oxetansilane der allgemeinen Formel (I) und Stereoisomere davon 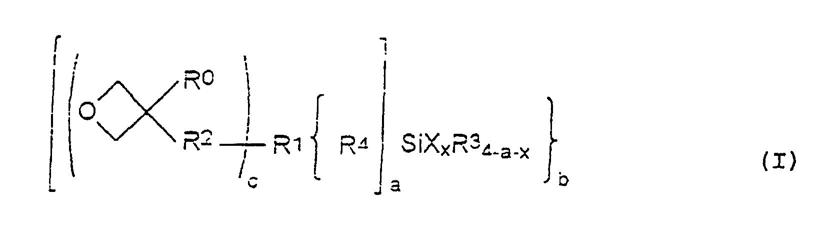 wobei die Variablen R⁰, R¹, R², R³, R⁴, R⁵, R⁶, X, Y, a, b, c, und x unabhängig voneinander die folgenden Bedeutungen haben:
R⁰ = Wasserstoff oder substituiertes oder unsubstituiertes C₁- bis C₁₀-Alkyl;
R¹ = entfällt oder substituiertes oder unsubstituiertes C₁- bis C₁₈-Alkylen, C₆- bis C₁₈-Arylen, C₇- bis C₁₈-Alkylenarylen oder -Arylenalkylen, wobei diese Reste durch mindestens eine Gruppe ausgewählt aus Ether-, Thioether-, Ester-, Carbonyl-, Amid- und Urethangruppe unterbrochen sein können;
R² = entfällt oder substituiertes oder unsubstituiertes C₁- bis C₁₈-Alkylen, C₆- bis C₁₈-Arylen, C₇- bis C₁₈-Alkylenarylen oder C₇- bis C₁₈- Arylenalkylen, wobei diese Reste durch mindestens eine Gruppe ausgewählt aus Ether-, Thioether-, Ester-, Thioester-, Carbonyl-, Amid- und Urethangruppe unterbrochen sein oder diese endständig tragen können;
R³ = entfällt oder substituiertes oder unsubstituiertes C₁- bis C₁₈-Alkyl, C₂- bis C₁₈-Alkenyl, C₆- bis C₁₈-Aryl, C₇- bis C₁₈-Alkylaryl oder C₇- bis C₁₈-Arylalkyl, wobei diese Reste durch mindestens eine Gruppe ausgewählt aus Ether-, Thioether-, Ester-, Carbonyl-, Amid- und Urethangruppe unterbrochen sein können;
R⁴ = substituiertes oder unsubstituiertes -CHR⁶-CHR⁶-S-R⁵-, -S-R⁵- oder -Y-CO-NH-R⁵- ;
R⁵ = substituiertes oder unsubstituiertes C₁- bis C₁₈-Alkylen, C₆- bis C₁₈-Arylen, C₆- bis C₁₈-Alkylenarylen oder C₆- bis C₁₈-Arylenalkylen, wobei diese Reste durch mindestens eine Gruppe ausgewählt aus Ether-, Thioether-, Ester-, Carbonyl-, Amid- und Urethangruppe unterbrochen sein können;
R⁶ = Wasserstoff oder substituiertes oder unsubstituiertes C₁- bis C₁₈-Alkyl oder C₆ bis C₁₀-Aryl;
X = eine hydrolysierbare Gruppe, nämlich Halogen, Hydroxy, Alkoxy oder Acyloxy;
Y = O oder S;
a = 1, 2 oder 3;
b = 1, 2 oder 3;
c = 1 bis 6; und
x = 1, 2 oder 3;
und mit der Maßgabe, daß
(i) a+x = 2, 3 oder 4
und
(ii) a und/oder b = 1.

2. Oxetansilane nach Anspruch 1, **dadurch gekennzeichnet, daß** mindestens eine der Variablen der Formel (I) unabhängig von den übrigen Variablen, die folgende Bedeutung hat:
R⁰ = Wasserstoff oder C₁- bis C₅-Alkyl;
R¹ = C₁- bis C₈-Alkylen, wobei diese Reste durch mindestens eine Gruppe ausgewählt aus Ether-, Thioether-, Ester- und Urethangruppe unterbrochen sein können;
R² = entfällt oder C₁- bis C₈-Alkylen, wobei diese Reste durch mindestens eine Gruppe ausgewählt aus Ether-, Thioether-, Ester-, Thioester, Carbonyl-, Amid- und Urethangruppe unterbrochen sein oder diese endständig tragen können;
R³ = entfällt oder Methyl, Ethyl oder Phenyl;
R⁴ = -S-R⁵- oder -Y-CO-NH-R⁵- ;
R⁵ = C₁- bis C₈-Alkylen, wobei diese Reste durch mindestens eine Gruppe ausgewählt aus Ether-, Thioether-, Ester-, Carbonyl-, Amid- und Urethangruppe unterbrochen sein können;
R⁶ = Wasserstoff oder C₁- bis C₅-Alkyl;
X = Methoxy, Ethoxy oder Chlor;
Y = O oder S;
a = 1;
b = 1;
c = 1 bis 6;
x = 2 oder 3; und/oder
a+x = 3.

3. Oxetansilane nach Anspruch 1 oder 2, **dadurch gekennzeichnet, daß** in der Formel (I) unabhängig von den übrigen Variablen, mindestens eine der Variablen a, b und c = 1.

4. Oxetansilane mit den Formeln:

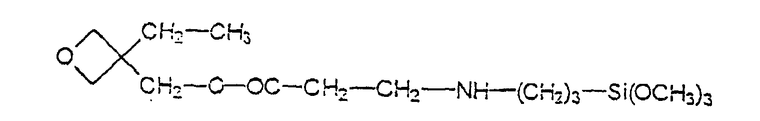

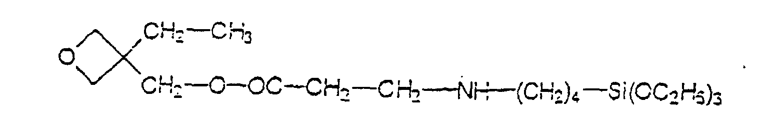

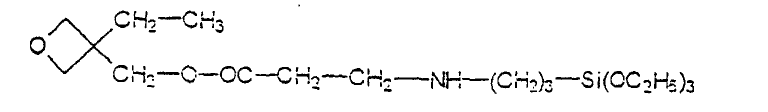

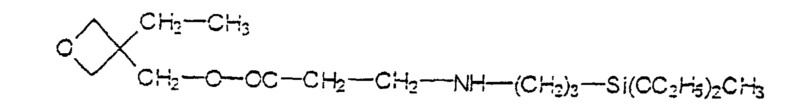

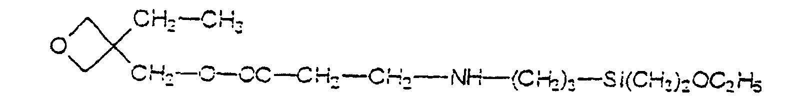

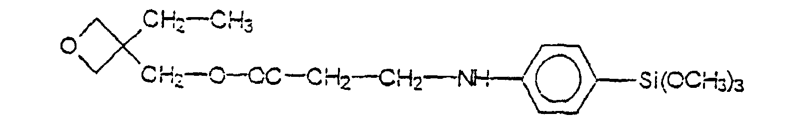

5. Polymerisierbare Kieselsäurekondensate der Oxetansilane gemäß einem der Ansprüche 1 bis 4, welche durch Hydrolyse und Kondensation der Oxetansilane (I) oder gemäß Anspruch 4, ggf. in Gegenwart weiterer hydrolysierbarer Verbindungen, erhältlich sind.

6. Polymerisate der Oxetansilane gemäß einem der Ansprüche 1 bis 4 oder der Kieselsäurekondensate gemäß Anspruch 5.

7. Zusammensetzungen mit Gehalt an den Oxetansilanen gemäß einem der Ansprüche 1 bis 4 oder an den Kieselsäurekondensaten gemäß Anspruch 5.

8. Zusammensetzungen nach Anspruch 7, die
(a) 5 bis 90, insbesondere 10 bis 70 Gew.-%, bezogen auf die Zusammensetzung, Kieselsäurekondensat gemäß Anspruch 5,
(b) 0 bis 80, insbesondere 0 bis 50 Gew.-%, bezogen auf die Zusammensetzung, Verdünnermonomer,
(c) 0,1 bis 5, insbesondere 0,2 bis 2,0 Gew.-%, bezogen auf die Zusammensetzung, Polymerisationsinitiator, und
(d) 0 bis 90, insbesondere 0 bis 80 Gew.-%, bezogen auf die Zusammensetzung, Füllstoffe
enthalten.

9. Verwendung von den Oxetansilanen gemäß einem der Ansprüche 1 bis 4, den Kieselsäurekondensaten nach Anspruch 5, den Polymerisaten nach Anspruch 6 oder den Zusammensetzungen nach Anspruch 7 oder 8 als Dentalmaterial oder als Bestandteil von Dentalmaterial.

## Claims

1. Hydrolysable and polymerizable oxetane silanes of the general formula (I) and stereoisomers thereof 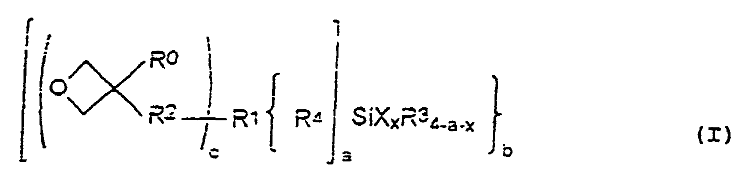 in which the variables R⁰, R¹, R², R³, R⁴, R⁵, R⁶, X, Y, a, b, c, and x independently of one another have the following meanings:
R⁰ = hydrogen or substituted or unsubstituted C₁ to C₁₀ alkyl;
R¹ = absent or substituted or unsubstituted C₁ to C₁₈ alkylene, C₆ to C₁₈ arylene, C₇ to C₁₈ alkylenearylene, or arylenealkylene, these radicals being able to be interrupted by at least one group selected from the group consisting of ether, thioether, ester, carbonyl, amide, and urethane groups;
R² = absent or substituted or unsubstituted C₁ to C₁₈ alkylene, C₆ to C₁₈ arylene, C₇ to C₁₈ alkylenearylene, or arylenealkylene, these radicals being able to be interrupted by at least one group selected from the group consisting of ether, thioether, ester, thioester, carbonyl, amide, and urethane groups or being able to bear these in the terminal position;
R³ = absent or substituted or unsubstituted C₁ to C₁₈ alkyl , C₂ to C₁₈ alkenyl, C₆ to C₁₈ aryl, C₇ to C₁₈ alkylaryl, or C₇ to C₁₈ arylalkyl, these radicals being able to be interrupted by at least one group selected from the group consisting of ether, thioether, ester, carbonyl, amide, and urethane groups;
R⁴ = substituted or unsubstituted -CHR⁶-CHR⁶-S-R⁵-, -S-R⁵- or -Y-CO-NH-R⁵-;
R⁵ = substituted or unsubstituted C₁ to C₁₈ alkylene, C₆ to C₁₈ arylene, C₆ to C₁₈ alkylenearylene, or C₆ to C₁₈ arylenealkylene, these radicals being able to be interrupted by at least one group selected from the group consisting of ether, thioether, ester, carbonyl, amide, and urethane groups;
R⁶ = hydrogen or substituted or unsubstituted C₁ to C₁₈ alkyl or C₆ to C₁₀ aryl;
X = a hydrolysable group, namely halogen, hydroxy, alkoxy, or acyloxy;
Y = O or S;
a = 1, 2, or 3;
b = 1, 2, or 3;
c = 1 to 6; and
x = 1, 2, or 3;
and with the proviso that
(i) a+x = 2, 3, or 4 and
(ii) a and/or b = 1.

2. Oxetane silanes according to Claim 1, **characterized in that** at least one of the variables of the formula (I) independently of the remaining variables has the following meaning:
R⁰ = hydrogen or C₁ to C₅ alkyl;
R¹ = C₁ to C₈ alkylene, these radicals being able to be interrupted by at least one group selected from the group consisting of ether, thioether, ester, and urethane groups;
R² = absent or is C₁ to C₈ alkylene, these radicals being able to be interrupted by at least one group selected from the group consisting of ether, thioether, ester, thioester, carbonyl, amide, and urethane groups or being able to bear these in the terminal position;
R³ = absent or is methyl, ethyl or phenyl;
R⁴ = -S-R⁵- or -Y-CO-NH-R⁵-;
R⁵ = C₁ to C₈ alkylene, these radicals being able to be interrupted by at least one group selected from the group consisting of ether, thioether, ester, carbonyl, amide, and urethane groups;
R⁶ = hydrogen or C₁ to C₅ alkyl;
X = methoxy, ethoxy or chloro;
Y = O or S;
a = 1;
b = 1;
c = 1 to 6;
x = 2 or 3; and/or
a+x = 3.

3. Oxetane silanes according to Claim 1 or 2, **characterized in that** in the formula (I), independently of the other variables, at least one of the variables a, b, and c = 1.

4. Oxetane silanes having the formulae:

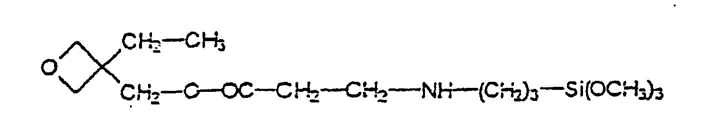

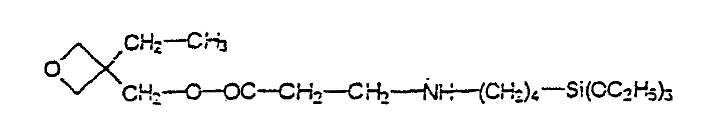

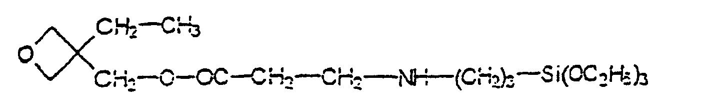

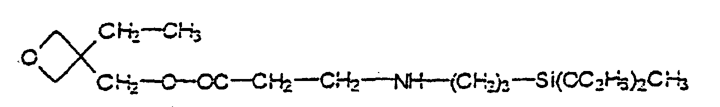

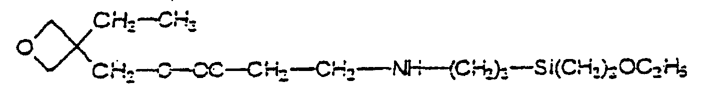

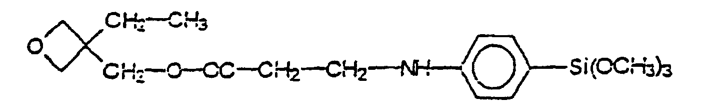

5. Polymerizable silicic acid condensates of the oxetane silanes according to one of Claims 1 to 4, which are obtainable by hydrolysis and condensation of the oxetane silanes (I) or according to Claim 4, in the presence if desired of further hydrolysable compounds.

6. Polymers of the oxetane silanes according to one of Claims 1 to 4 or of the silicic acid condensates according to Claim 5.

7. Compositions comprising the oxetane silanes according to one of Claims 1 to 4 or the silicic acid condensates according to Claim 5.

8. Compositions according to Claim 7 containing
(a) from 5 to 90% by weight, in particular from 10 to 70% by weight, based on the composition, of silicic acid condensate according to Claim 5,
(b) from 0 to 80% by weight, in particular from 0 to 50% by weight, based on the composition, of diluent monomer,
(c) 0.1 to 5% by weight, in particular from 0.2 to 2.0% by weight, based on the composition, of polymerization initiators, and
(d) from 0 to 90% by weight, in particular from 0 to 80% by weight, based on the composition, of fillers.

9. Use of the oxetane silanes according to one of Claims 1 to 4, the silicic acid condensates according to Claim 5, the polymers according to Claim 6 or the compositions according to Claim 7 or 8 as dental material or as a constituent of dental material.

## Revendications

1. Oxetanesilanes hydrolysables et polymérisables de la formule générale (I) et stéréoisomères de ceux-ci : 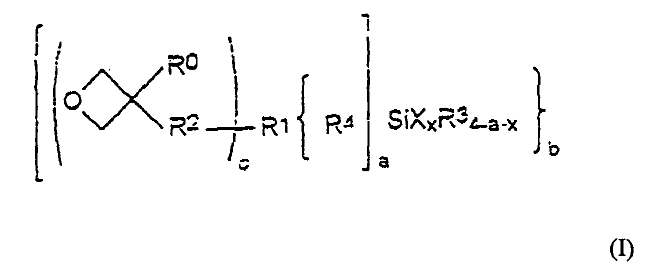 dans laquelle les variables R⁰, R¹, R², R³, R⁴, R⁵, R⁶, X, Y, a, b, c et x, indépendamment l'une de l'autre, ont les significations :
R⁰ =hydrogène ou alkyle en C₁ à C₁₀ substitué ou non substitué ;
R¹ =absent, ou alkylène en C₁ à C₁₈, arylène en C₆ à C₁₈, alkylène-arylène ou arylènealkylène en C₇ à C₁₈, substitués ou non substitués, ces résidus pouvant être interrompus par au moins un groupe choisi dans le groupe éther, thioéther, ester, carbonyle, amide et uréthane ;
R² =absent, ou alkylène en C₁ à C₁₈, arylène en C₆ à C₁₈, alkylène-arylène en C₇ à C₁₈ ou arylène-alkylène en C₇ à C₁₈, substitués ou non substitués, ces résidus pouvant être interrompus par au moins un groupe choisi dans le groupe éther, thioéther, ester, thioester, carbonyle, amide et uréthane ou pouvant porter ceux-ci en extrémité ;
R³ =absent ou alkyle en C₁ à C₁₈, alkényle en C₂ à C₁₈, aryle en C₆ à C₁₈, alkylaryle en C₇ à C₁₈, ou arylalkyle en C₇ à C₁₈, substitués ou non substitués, ces résidus pouvant être interrompus par au moins un groupe choisi dans le groupe éther, thioéther, ester, carbonyle, amide et uréthane ;
R⁴ =-CHR⁶-CHR⁶-S-R⁵-, -S-R⁵- ou -Y-CO-NH-R⁵- substitué ou non substitué ;
R⁵ =alkylène en C₁ à C₁₈, arylène en C₆ à C₁₈, alkylène-arylène en C₆ à C₁₈ ou arylène-alkylène en C₆ à C₁₈, substitués ou non substitués, ces résidus pouvant être interrompus par au moins un groupe choisi dans le groupe éther, thioéther, ester, carbonyle, amide et uréthane ;
R⁶ =hydrogène ou alkyle en C₁ à C₁₈ ou aryle en C₆ à C₁₀, substitué ou non substitué ;
X =un groupe hydrolysable, à savoir halogène, hydroxy, alkoxy ou acyloxy ;
Y =O ou S ;
a =1, 2 ou 3 ;
b =1, 2 ou 3 ;
c =1 à 6, et
x =1, 2 ou 3 ;
et à la condition que
(i) a+x = 2, 3 ou 4
et
(ii) a et/ou b = 1.

2. Oxetanesilanes selon la revendication 1, **caractérisés en ce qu'**au moins l'une des variables de la formule (I) a, indépendamment l'une de l'autre, la signification suivante :
R⁰ =hydrogène ou alkyle en C₁ à C₅ ;
R¹ =alkylène en C₁ à C₈, ces résidus pouvant être interrompus par au moins un groupe choisi dans le groupe éther, thioéther, ester et uréthane ;
R² =absent ou aikylène en C₁ à C₈, ces résidus pouvant être interrompus par au moins un groupe choisi dans le groupe éther, thioéther, ester, thioester, carbonyle, amide et uréthane ou pouvant porter ceux-ci en extrémité ;
R³ =absent ou méthyle, éthyle ou phényle ;
R⁴ =-S-R⁵- ou -Y-CO-NH-R⁵ ;
R⁵ =alkylène en C₁ à C₈, ces résidus pouvant être interrompus par au moins un groupe choisi dans le groupe éther, thioéther, ester, carbonyle, amide et uréthane ;
R⁶ =hydrogène ou alkyle en C₁ à C₅ ;
X =méthoxy, éthoxy ou chlore ;
Y =O ou S ;
a =1 ;
b =1 ;
c =1 à 6 ;
x =2 ou 3, et/ou
a+x =3.

3. Oxetanesilanes selon la revendication 1 ou 2, **caractérisés en ce que**, dans la formule (I), indépendamment des autres variables, au moins l'une des variables a, b et c = 1.

4. Oxetanesilanes ayant les formules :

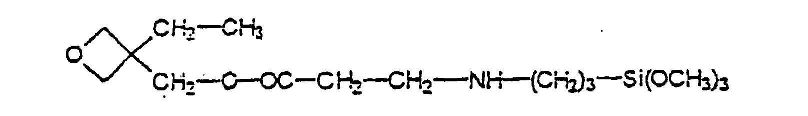

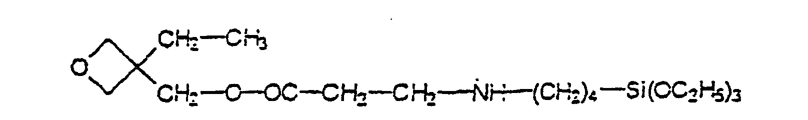

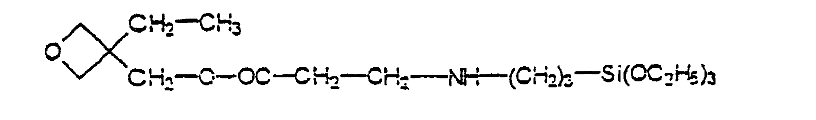

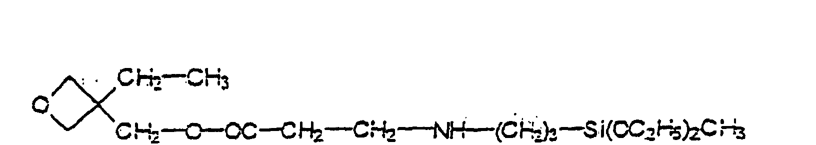

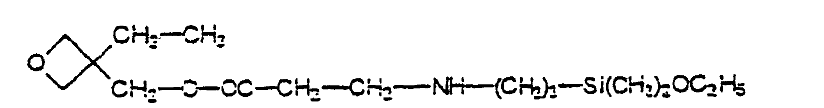

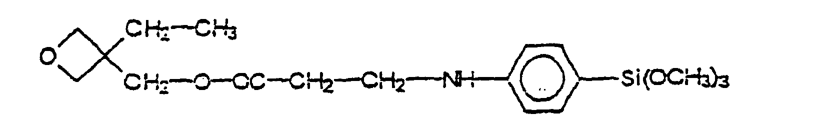

5. Condensats d'acide silique polymérisables des oxetanesilanes selon l'une des revendications 1 à 4, qui sont susceptibles d'être obtenus par hydrolyse et condensation des oxetanesilanes (I) ou selon la revendication 4, éventuellement en présence d'autres composéss hydrolysables.

6. Polymérisats des oxetanesilanes selon l'une des revendications 1 à 4 ou des condensats d'acide silique selon la revendication 5.

7. Compositions ayant une teneur en oxetanesilanes selon l'une des revendications 1 à 4 ou en condensats d'acide silique selon la revendication 5.

8. Compositions selon la revendication 7 qui contiennent :
(a) 5 à 90, en particulier 10 à 70 % en poids, par rapport à la composition, de condensat d'acide silique selon la revendication 5,
(b) 0 à 80, en particulier 0 à 50 % en poids, par rapport à la composition, de monomère de diluant,
(c) 0,1 à 5, en particulier 0,2 à 2,0 % en poids, par rapport à la composition, d'initiateur de polymérisation et
(d) 0 à 90, en particulier 0 à 80 % en poids, par rapport à la composition, de matières de charge.

9. Utilisation des oxetanesilanes selon l'une des revendications 1 à 4, des condensats d'acide silique selon la revendication 5, des polymérisats selon la revendication 6 ou des compositions selon la revendication 7 ou 8 comme matériau dentaire ou composant de matériau dentaire.
